# EUROPEAN PATENT APPLICATION

(11) **EP 4 586 259 A1**
(43) Date of publication of application: **16.07.2025**
(21) Application number: 24223491.2
(22) Date of filing: 27.12.2024
(51) Int. Cl.: G16H 10/20, G16H 10/60

(54) **METHOD AND SYSTEM FOR AUTOMATED CLINICAL EVENT AND ENDPOINT ADJUDICATION**

(30) Priority: 15.01.2024 IN 202421002945
(71) Applicant: Tata Consultancy Services Limited, Maharashtra (IN)
(72) Inventor: DAS, Saurabh, 400076 Mumbai - Maharashtra (IN); VYAS, Niraj, 400076 Mumbai - Maharashtra (IN); PACHISIA, Ashutosh, 400076 Mumbai - Maharashtra (IN); GUERCHICOFF, Alejandra, New Jersey, 08837 (US); CHOUDHARY, Karam Veer Sing, Texas, 77377 (US); KADAM, Rohit, 411057 Pune - Maharashtra (IN); PANCHAL, Niketan, 400076 Mumbai - Maharashtra (IN); CHATURVEDI, Prashant, 500034 Hyderabad - Telangana (IN)
(74) Representative: Goddar, Heinz J.

(57) **Abstract**

Traditional adjudication process is expensive, time consuming and requires experts for reviewing events and endpoints. Thus, embodiments of present disclosure provide a method and system for automated clinical event adjudication. Input data required for clinical event adjudication including patient clinical information and clinical trial master documents are obtained. Then, adjudication rules are generated from the clinical trial master documents based on which adjudicable clinical events and endpoints are identified from the patient clinical information. Further, processing models are configured and executed to get a first result of clinical event adjudication. A second result of clinical event adjudication is obtained by processing the adjudicable events and endpoints using a pre-trained real world evidence inference model. Then, a weighted average technique is applied on the first result and the second result to obtain a final result of clinical event adjudication based on which an adjudication report is generated.

## Description

### CROSS-REFERENCE TO RELATED APPLICATIONS AND PRIORITY

The present application claims priority to Indian application no. 202421002945, filed on January 15, 2024.

### TECHNICAL FIELD

The disclosure herein generally relates to event and endpoint adjudication, and, more particularly, to a method and system for automated clinical event and endpoint adjudication.

### BACKGROUND

Event and endpoint adjudication is a standard process for the assessment of safety and efficacy of pharmacologic or device therapies in clinical trials wherein an independent, and most of the time, blinded expert committee reviews clinical events that occur during the trial. These are assessed and adjudicated against a set of pre-defined criteria and supporting clinical documentation. Endpoint adjudication is managed in several ways, but mostly via electronic system which provides accountability and documentation of the entire process. The Clinical Event Committee (CEC) is responsible for the event adjudication process. It comprises of experts in the domain who come together to analyze the clinical trial data. The adjudication process is entirely dependent on the CEC which makes the process manual and prone to errors and human bias. Also, the process is time consuming and the delays in adjudication frequently impact the course of the clinical trial. Manual adjudication process presents inconsistencies due to human based intervention. Delays in adjudication can put the clinical study at risk of being suspended. It is challenging to automate the process of event adjudication since the data is huge and in silos. It is difficult to process the data with consistency following clinical trial adverse event definitions and adjudication guidelines. Currently, there are no solutions to generate clean, trustable, and consistent adjudication data and to use historical data to evaluate and monitoring adverse events prospectively. Currently available safety signals tools use outdated data to identified potential risk even during the study due to data access or process inefficiencies.

### SUMMARY

Embodiments of the present disclosure present technological improvements as solutions to one or more of the above-mentioned technical problems recognized by the inventors in conventional systems. For example, in one embodiment, a method for automated clinical event and endpoint adjudication is provided. The method includes obtaining an input data related to one or more clinical trials and clinical event adjudication. The input data comprises: (i) patient clinical information, and (ii) clinical trial master documents. Further, the method includes generating one or more adjudication rules from the clinical trial master documents and identifying one or more adjudicable clinical events and one or more endpoints from the patient clinical information based on the one or more adjudication rules. The method further includes determining a configuration of a plurality of processing models for processing the one or more adjudicable events and the one or more endpoints and executing the plurality of processing models according to the determined configuration using the patient clinical information to obtain a first result of the clinical event adjudication. Further, the method includes obtaining a second result of the clinical event adjudication by processing the one or more adjudicable events and the one or more endpoints using a pre-trained real world evidence inference model and applying a weighted average technique on the first result of the clinical event adjudication and the second result of the clinical event adjudication to obtain a final result of the clinical event adjudication. Furthermore, the method includes generating an adjudication report based on the final result of the clinical event adjudication and one or more adjudication forms comprised in the clinical trial master documents.

In another aspect, a system for automated clinical event and endpoint adjudication is provided. The system includes a memory storing instructions; one or more communication interfaces; and one or more hardware processors coupled to the memory via the one or more communication interfaces, wherein the one or more hardware processors are configured by the instructions to: obtain an input data related to one or more clinical trials and clinical event adjudication. The input data comprises: (i) patient clinical information, and (ii) clinical trial master documents. Further, the one or more hardware processors are configured to generate one or more adjudication rules from the clinical trial master documents and identify one or more adjudicable clinical events and one or more endpoints from the patient clinical information based on the one or more adjudication rules. The one or more hardware processors are further configured to determine a configuration of a plurality of processing models for processing the one or more adjudicable events and the one or more endpoints and execute the plurality of processing models according to the determined configuration using the patient clinical information to obtain a first result of the clinical event adjudication. Further, the one or more hardware processors are configured to obtain a second result of the clinical event adjudication by processing the one or more adjudicable events and the one or more endpoints using a pre-trained real world evidence inference model and apply a weighted average technique on the first result of the clinical event adjudication and the second result of the clinical event adjudication to obtain a final result of the clinical event adjudication. Furthermore, the one or more hardware processors are configured to generate an adjudication report based on the final result of the clinical event adjudication and one or more adjudication forms comprised in the clinical trial master documents.

In yet another aspect, there are provided one or more non-transitory machine-readable information storage mediums comprising one or more instructions which when executed by one or more hardware processors cause a method for automated clinical event and endpoint adjudication. The method includes obtaining an input data related to one or more clinical trials and clinical event adjudication. The input data comprises: (i) patient clinical information, and (ii) clinical trial master documents. Further, the method includes generating one or more adjudication rules from the clinical trial master documents and identifying one or more adjudicable clinical events and one or more endpoints from the patient clinical information based on the one or more adjudication rules. The method further includes determining a configuration of a plurality of processing models for processing the one or more adjudicable events and the one or more endpoints and executing the plurality of processing models according to the determined configuration using the patient clinical information to obtain a first result of the clinical event adjudication. Further, the method includes obtaining a second result of the clinical event adjudication by processing the one or more adjudicable events and the one or more endpoints using a pre-trained real world evidence inference model and applying a weighted average technique on the first result of the clinical event adjudication and the second result of the clinical event adjudication to obtain a final result of the clinical event adjudication. Furthermore, the method includes generating an adjudication report based on the final result of the clinical event adjudication and one or more adjudication forms comprised in the clinical trial master documents.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for automated clinical event and endpoint adjudication, according to some embodiments of the present disclosure.
FIGS. 2A and 2B, collectively referred as FIG. 2, illustrates a flow diagram of a method of automated clinical event and endpoint adjudication, according to some embodiments of the present disclosure.
FIG. 3 is an architecture diagram illustrating automated clinical event and endpoint adjudication system implemented by system of FIG. 1, according to some embodiments of the present disclosure.
FIG. 4 illustrates a traditional clinical event adjudication process.
FIG. 5 illustrates an example configuration of a plurality of processing models, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

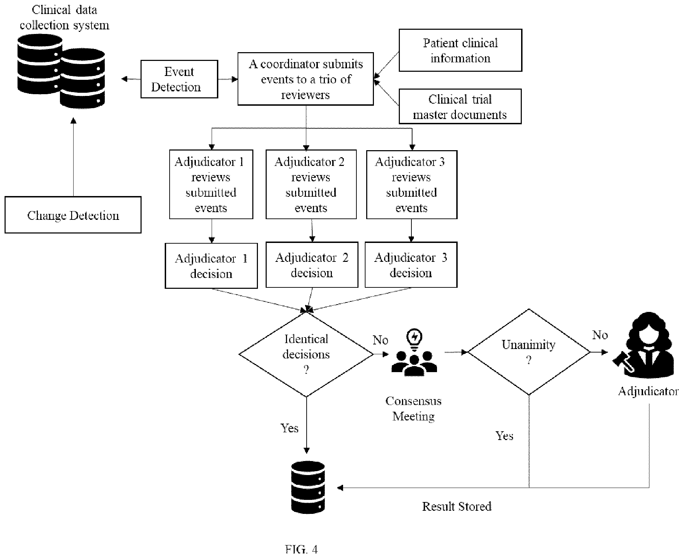

Clinical event and endpoint adjudication is an important aspect of clinical study. As illustrated in FIG. 4, the traditional (alternatively referred as conventional) clinical event adjudication process involves obtaining patient clinical information and clinical trial master documents. Events are manually identified from the obtained data and submitted to a panel of 3 or more adjudicators. Each adjudicator reviews the events independently and decides whether they are adjudicable or not. If all the adjudicators arrive at a same clinical adjudication result then the result is stored in a database. Otherwise, a consensus meeting is held among the adjudicators in the presence of a coordinator. If the adjudicators arrive at a common conclusion then the result is stored in the database. In case their decision is not unanimous, then the events are taken to another adjudicator to arrive at a final decision. This modality is expensive, time consuming and requires experts for reviewing the events. It is also prone to human errors.

In order to overcome the drawbacks of traditional process, embodiments of present disclosure provide a method and system for automated clinical event adjudication. The system obtains input data required for clinical event adjudication including patient clinical information and clinical trial master documents. Then, adjudication rules are generated from the clinical trial master documents based on which adjudicable clinical events and endpoints are identified from the patient clinical information. Further, processing models for processing the adjudicable events and endpoints are configured and executed to get a first result of clinical event adjudication. A second result of clinical event adjudication is obtained by processing the adjudicable events and endpoints using a pre-trained real world evidence inference model. Then, a weighted average technique is applied on the first result and the second result to obtain a final result of clinical event adjudication based on which an adjudication report is generated. Since the method is completely automated, clinical event adjudication process is completed in a shorter time as compared to traditional process.

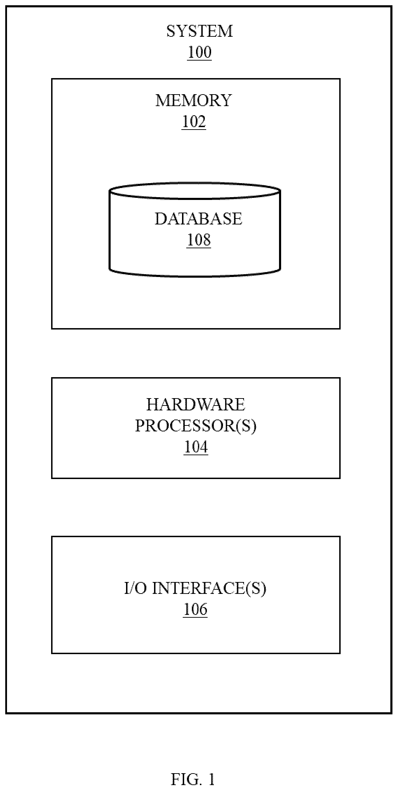

Referring now to the drawings, and more particularly to FIGS. 1, 2, 3 and 5, where similar reference characters denote corresponding features consistently throughout the figures, there are shown preferred embodiments and these embodiments are described in the context of the following exemplary system and/or method.

FIG. 1 illustrates an exemplary block diagram of a system for automated clinical event adjudication, according to some embodiments of the present disclosure. In an embodiment, the system 100 includes one or more processors (104), communication interface device(s) (106) or Input/Output (I/O) interface(s) (106) or user interface (106), and one or more data storage devices or memory (102) operatively coupled to the one or more processors (104). The one or more processors (104) that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

The I/O interface device(s) (106) can include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface device(s) (106) receives patient clinical information and clinical trial master documents as input data and provides an adjudication report as output.

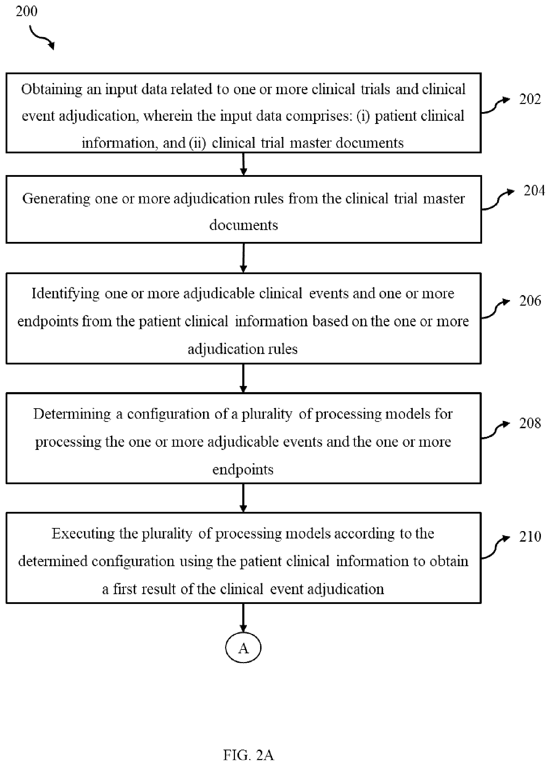

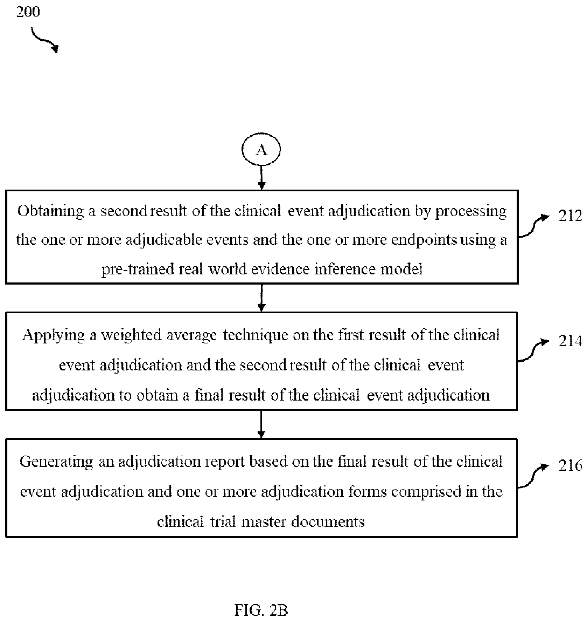

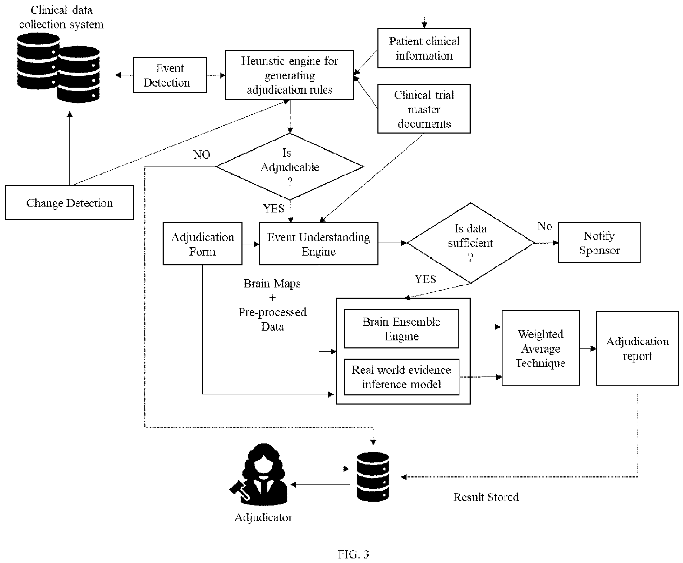

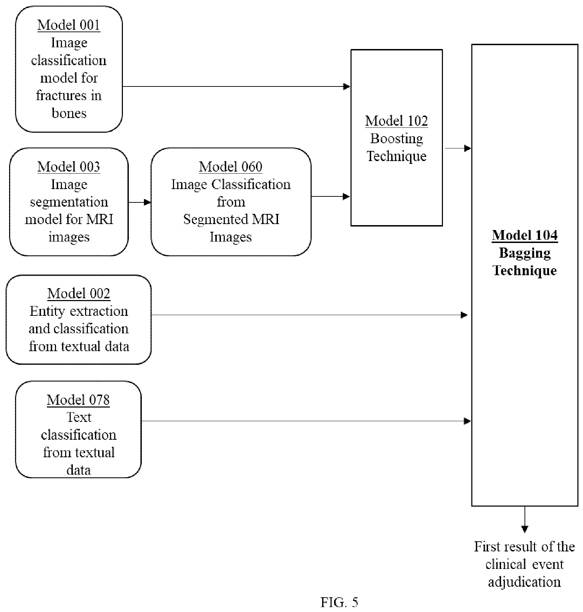

The memory (102) may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. The database 108 may store information but not limited to information associated with at least one of: input data, processing models, decision matrix, pre-trained real world evidence inference model and so on. Further, the database 108 stores information pertaining to inputs fed to the system 100 and/or outputs generated by the system (e.g., at each stage), specific to the methodology described herein. Functions of the components of system 100 are explained in conjunction with flow diagram depicted in FIG. 2, architecture diagram illustrated in FIG. 3 for automated clinical event and endpoint adjudication and an example configuration illustrated in FIG. 5.

In an embodiment, the system 100 comprises one or more data storage devices or the memory (102) operatively coupled to the processor(s) (104) and is configured to store instructions for execution of steps of the method (200) depicted in FIG. 2 by the processor(s) or one or more hardware processors (104). The steps of the method of the present disclosure will now be explained with reference to the components or blocks of the system 100 as depicted in FIG. 1, the steps of flow diagrams as depicted in FIG. 2, architecture diagram illustrated in the FIG. 3 and an example configuration illustrated in FIG. 5. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

FIGS. 2A and 2B, collectively referred as FIG. 2, illustrates a flow diagram of a method (200) of automated clinical event and endpoint adjudication. It is explained in conjunction with architecture diagram of FIG. 3 for automated clinical event and endpoint adjudication. At step 202 of the method 200, the one or more hardware processors 104 are configured to obtain an input data related to one or more clinical trials and clinical event or endpoint adjudication. The input data comprises: (i) patient clinical information, and (ii) clinical trial master documents. The patient clinical information is obtained from at least one of: (i) one or more source documents, and (ii) an input feed from one or more clinical data collection systems. The clinical trial master documents comprise a Clinical Event Committee (CEC) charter, a clinical protocol, and one or more adjudication forms. The CEC charter outlines one or more procedures, rules, and guidelines for conducting the clinical event adjudication. The clinical protocol describes one or more objectives, design, methodology, statistical considerations, and aspects related to organization of the one or more clinical trials. In some cases when the CEC charter may not be available, the clinical protocol outlines one or more procedures, rules, and guidelines for conducting the clinical event adjudication.

An example clinical protocol is given below-

### PROTOCOL

Primary Endpoints:
- All causes of mortality at 1 year of enrollment.

Secondary Endpoints:
- Death (within 6 months from dosage)
- Stroke/ TIA (through 24 months from dosage)
- Myocardial Infarction (through 30 days from dosage)

An example CEC charter is given below:
Events to be adjudicated: As per Clinical Protocol and CEC Charter the following were the safety events to be adjudicated-

Site-reported and suspected events:
- Death (within 6 months from dosage)
- Stroke/ TIA (through 24 months from dosage)
- Myocardial Infarction (through 30 days from dosage)
- All hospitalizations - all causes (through 24 months)

Adjudication procedure:
The clinical protocol or the CEC Charter (CEC Manual of Operations) describes- (1) the adjudicator selection as well as the roles and responsibilities of each CEC member, (2) the roles and responsibilities of external providers of CEC services, (3) determines whether the site reported endpoints meet the protocol-specified criteria and definitions for an event to be selected for adjudication (4) the adjudication criteria and clinical definitions to be apply to each event when assess it, and (3) the evaluation procedure the CEC will use in reviewing the trial endpoint/ events to be adjudicated. The CEC charter is updated with each revision of clinical protocol only if it impacts the CEC process or clinical endpoint definition(s) is revised.

### DOCUMENT REQUIREMENTS

### Required Data for CEC Adjudication of Events

Each event shall include the information (but is not limited to or required):
All clinical protocol and/or CEC Charter are defined and available documents related to the potential clinical endpoint or event to be adjudicated.

Once the input data is received, the clinical trial master documents maybe compared with one or more previously obtained clinical trial master documents to identify if there are older versions of the clinical trial master documents. Upon identification of the older versions of the clinical trial master documents, one or more changes in the clinical trial master documents are identified and the step 204 is performed only for the one or more changes. At the step 204 of the method 200, the one or more hardware processors 104 are configured to generate one or more adjudication rules from the clinical trial master documents. This step is performed by heuristic engine illustrated in FIG. 3. The one or more adjudication rules are generated by first identifying and extracting a plurality of relevant sections from the CEC charter and the clinical protocol. The plurality of relevant sections are related to one or more adjudicable events and one or more endpoints. Then, a plurality of entities and a plurality of relations between the plurality of entities are extracted from the plurality of relevant sections. Finally, the plurality of entities and the plurality of relations are converted into one or more logical statements, wherein the one or more logical statements form the one or more adjudication rules.

For example, the adjudication rules generated at step 204 for the above example clinical protocol and CEC charter are:
1. If Event=='Death' & current date - dosage date < 6 months
   Then Adjudication Eligible= True
2. If Event ==' Stroke' & current date - dosage date < 24 months
   Then Adjudication Eligible= True
3. If Event=='Myocardial Infarction' & current date - dosage date <30 days Then Adjudication Eligible= True

Once the one or more adjudication rules are generated, at step 206 of the method 200, the one or more hardware processors 104 are configured to identify one or more adjudicable clinical events and one or more endpoints from the patient clinical information based on the one or more adjudication rules. Clinical event is an adverse clinical situation such as myocardial infarction while endpoint refers to an analyzed parameter such as change from baseline at 6 weeks. Both of them are identified for performing adjudication. Further, at step 208 of the method 200, the one or more hardware processors 104 are configured to determine a configuration of a plurality of processing models for processing the one or more adjudicable events and the one or more endpoints. The steps 206 and 208 are performed by event understanding engine illustrated in FIG. 3. Initially, the one or more hardware processors 104 determine if the patient clinical information is sufficient or insufficient in accordance with the clinical trial master documents. In an embodiment, the data sufficiency check is performed using Natural Language Processing techniques. For example, the data sufficiency check involves checking if there are any missing values, data required in specific fields is in proper data type or not (if data is required in float values, but is present as string then it is incorrect. The data sufficiency check may also involve checking the unstructured textual patient clinical information for medical condition, drug, dosage, frequency, and other such conditions using medical named entity recognition techniques. If the information is sufficient, then further steps are performed. Otherwise, the system 100 notifies a user that the data is insufficient and obtains required data from the user. Suppose the user doesn't provide the required data even after multiple reminders, the system proceeds with further steps and marks the required data as permanently missing in the system. Once the data sufficiency check is done, data related to the one or more adjudicable events and the one or more endpoints are filtered from the patient clinical information according to the clinical trial master documents. The filtered data is then pre-processed using techniques such as data anonymization, language standardization, event labeling, unit or format standardization, converting files or images in the filtered data to proper format, summarization of patient clinical information, versioning Time series data, if available (involves conversion of data based on dates), validation of filtered data, validating time of transmission of data, checking accuracy of patient clinical information, and so on. Once the filtered data is pre-processed, the plurality of processing models are selected using a pre-defined decision matrix based on a data type of the filtered data and a medical field in which the one or more clinical trials are conducted. An example decision matrix is given in table 1. It is prepared by domain expert and is configurable. The plurality of processing models comprise algorithms, Artificial Intelligence (AI) or Machine Learning (ML) models, inference engines etc. stored in the database 108 (alternatively referred as knowledge base or knowledge farm). For example, Model 001 is an image classification model for fractures in bones, model 002 is entity extraction and classification model, model 003 is an image segmentation model for MRI images, model 004 is a regression model to predict the values from given features etc. The processing models are pre-trained and stored in the database. They may be retrained periodically using the input data to the system 100.

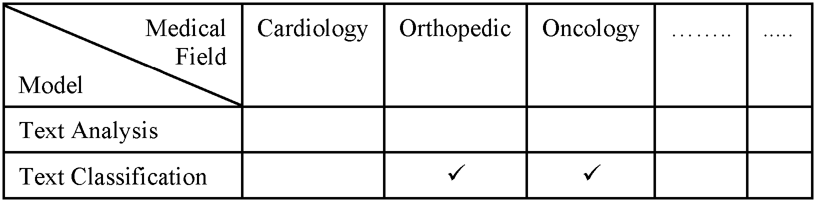

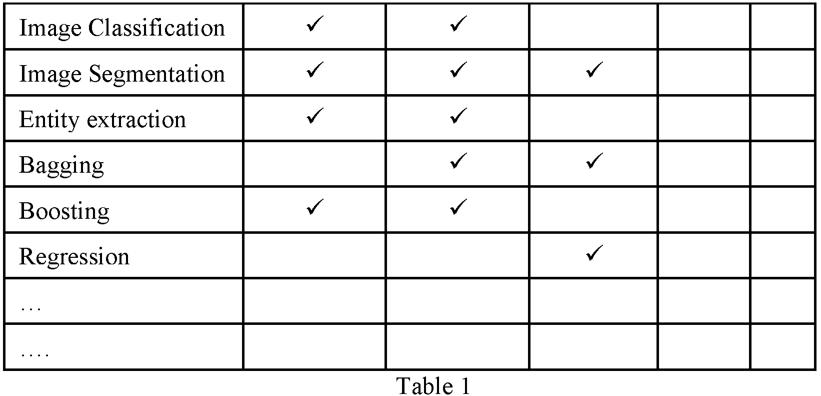

Once the plurality of processing models are selected, a vector indicating configuration of executing the plurality of processing models for the clinical event and endpoint adjudication is generated. Table 2 illustrates an example configuration. The processing models and their interconnection associated with the example configuration is illustrated in FIG. 5. In table 2, entry in first row and first column defines how the individual processing models are connected to each other. 0001 indicates that the models are connected sequentially. The entry in first row and second column represent the type of output. 0006 indicates that the processing models in this configuration give a single output (Yes/No) along with confidence score. Subsequent rows contain information about the models present in each layer. The models 001,003,002,0078 - are in first layer (0000 is padded after model numbers if there are extra models in other layers). Model 060 is directly below model 003 (in the same column) to indicate that output of 003 is given as input to 060. The same configuration is illustrated in FIG. 5, wherein the models 001,003,002,0078 are in first layer and can be executed in parallel. In the next layer, output of model 0003 is given as input to model 0060. In the third layer, outputs from model 0001 and model 0060 are fed into model 0102. Finally, at the last layer outputs from the models 0102, 0002 and 0078 are fed into the model 0104.

**Table 2**

| | | | | | |
|---|---|---|---|---|---|
| 0001 | 0006 | 0000 | 0000 | 0000 | 0000 |
| 0001 | 0003 | 0002 | 0078 | 0000 | 0000 |
| 0000 | 0060 | 0000 | 0000 | 0000 | 0000 |
| 0102 | 0102 | 0000 | 0000 | 0000 | 0000 |
| 0104 | 0104 | 0104 | 0104 | 0000 | 0000 |

Once the configuration of the plurality of processing models is determined, at step 210 of the method 200, the one or more hardware processors 104 are configured to execute the plurality of processing models (via brain ensemble engine illustrated in FIG. 3) according to the determined configuration using the patient clinical information to obtain a first result of the clinical event adjudication. Further, at step 212 of the method 200, the one or more hardware processors 104 are configured to obtain a second result of the clinical event adjudication by processing the one or more adjudicable events and the one or more endpoints using a pre-trained real world evidence inference model. The real world evidence inference model is trained to predict or classify adjudication result from the historical data of adjudicated cases. It is helpful in scenarios where the patient clinical information available is less than a tolerance limit. The real world evidence inference model is executed with available data to generate the second result based on previous result of similar events and endpoints.

In order to remove biasness in the adjudication result, at step 214 of the method 200, the one or more hardware processors 104 are configured to apply a weighted average technique on the first result of the clinical event adjudication and the second result of the clinical event adjudication. The weighted average technique is applied based on one or more weights determined using a historical data and a medical knowledge. For example, if many similar events or endpoints have been reported in the past, a higher weight will be assigned to the output from real world evidence inference model and vice versa. Finally, at step 216 of the method 200, the one or more hardware processors 104 are configured to generate an adjudication report based on the final result of the clinical event adjudication and one or more adjudication forms comprised in the clinical trial master documents. The adjudication report comprises filled one or more adjudication forms.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200), comprising:
obtaining (202), via one or more hardware processors, an input data related to one or more clinical trials and clinical event adjudication, wherein the input data comprises: i) patient clinical information, and ii) clinical trial master documents;
generating (204), via the one or more hardware processors, one or more adjudication rules from the clinical trial master documents;
identifying (206), via the one or more hardware processors, one or more adjudicable clinical events and one or more endpoints from the patient clinical information based on the one or more adjudication rules;
determining (208), via the one or more hardware processors, a configuration of a plurality of processing models for processing the one or more adjudicable events and the one or more endpoints;
executing (210), via the one or more hardware processors, the plurality of processing models according to the determined configuration using the patient clinical information to obtain a first result of the clinical event adjudication;
obtaining (212), via the one or more hardware processors, a second result of the clinical event adjudication by processing the one or more adjudicable events and the one or more endpoints using a pre-trained real world evidence inference model;
applying (214), via the one or more hardware processors, a weighted average technique on the first result of the clinical event adjudication and the second result of the clinical event adjudication to obtain a final result of the clinical event adjudication; and
generating (216), via the one or more hardware processors, an adjudication report based on the final result of the clinical event adjudication and one or more adjudication forms comprised in the clinical trial master documents.

2. The method as claimed in claim 1, wherein the patient clinical information is obtained from at least one of: (i) one or more source documents, and (ii) an input feed from one or more clinical data collection systems, and wherein the clinical trial master documents comprise a Clinical Event Committee (CEC) charter, a clinical protocol, and the one or more adjudication forms, wherein the CEC charter outlines one or more procedures, rules, and guidelines for conducting the clinical event adjudication, and wherein the clinical protocol describes one or more objectives, design, methodology, statistical considerations, and aspects related to organization of the one or more clinical trials.

3. The method as claimed in claim 2, wherein generating the one or more adjudication rules from the clinical trial master documents comprises:
identifying and extracting a plurality of relevant sections from the CEC charter and the clinical protocol, wherein the plurality of relevant sections are related to the one or more adjudicable events and the one or more endpoints;
extracting a plurality of entities in the plurality of relevant sections;
extracting a plurality of relations between the plurality of entities from the plurality of relevant sections; and
converting the plurality of entities and the plurality of relations into one or more logical statements, wherein the one or more logical statements form the one or more adjudication rules.

4. The method as claimed in claim 1, wherein configuring the plurality of processing models for processing the one or more adjudicable events and the one or more endpoints comprises:
filtering data related to the one or more adjudicable events and the one or more endpoints from the patient clinical information according to the clinical trial master documents;
pre-processing the filtered data;
selecting the plurality of processing models using a pre-defined decision matrix based on a data type of the filtered data and a medical field in which the one or more clinical trials are conducted; and
generating a vector indicating configuration of executing the plurality of processing models for the clinical event and endpoint adjudication.

5. The method as claimed in claim 4, comprising:
determining if the patient clinical information is sufficient or insufficient in accordance with the clinical trial master documents prior to the step of filtering data; and
performing one of: i) proceeding with the step of filtering data if the patient clinical information is sufficient, and ii) obtaining required data if the patient clinical information is insufficient, wherein if the required data is not available performing:
proceeding with the step of filtering data; and
marking the required data as permanently missing.

6. The method as claimed in claim 1, wherein the weighted average technique is applied based on one or more weights determined using a historical data and a medical knowledge.

7. The method as claimed in claim 1, wherein the clinical trial master documents obtained as input data are compared with one or more previously obtained clinical trial master documents to identify if there are older versions of the clinical trial master documents, wherein upon identification of the older versions of the clinical trial master documents, performing:
identifying one or more changes in the clinical trial master documents; and
generating the adjudication rules associated with the one or more changes.

8. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
obtain an input data related to one or more clinical trials and clinical event adjudication, wherein the input data comprises: i) patient clinical information, and ii) clinical trial master documents;
generate one or more adjudication rules from the clinical trial master documents;
identify one or more adjudicable clinical events and one or more endpoints from the patient clinical information based on the one or more adjudication rules;
determine a configuration of a plurality of processing models for processing the one or more adjudicable events and the one or more endpoints;
execute the plurality of processing models according to the determined configuration using the patient clinical information to obtain a first result of the clinical event adjudication;
obtain a second result of the clinical event adjudication by processing the one or more adjudicable events and the one or more endpoints using a pre-trained real world evidence inference model;
apply a weighted average technique on the first result of the clinical event adjudication and the second result of the clinical event adjudication to obtain a final result of the clinical event adjudication; and
generate an adjudication report based on the final result of the clinical event adjudication and one or more adjudication forms comprised in the clinical trial master documents.

9. The system as claimed in claim 8, wherein the patient clinical information is obtained from at least one of: i) one or more source documents, and ii) an input feed from one or more clinical data collection systems, and wherein the clinical trial master documents comprise a Clinical Event Committee (CEC) charter, a clinical protocol, and the one or more adjudication forms, wherein the CEC charter outlines one or more procedures, rules, and guidelines for conducting the clinical event adjudication, and wherein the clinical protocol describes one or more objectives, design, methodology, statistical considerations, and aspects related to organization of the one or more clinical trials.

10. The system as claimed in claim 9, wherein generating the one or more adjudication rules from the clinical trial master documents comprises:
identifying and extracting a plurality of relevant sections from the CEC charter and the clinical protocol, wherein the plurality of relevant sections are related to the one or more adjudicable events and the one or more endpoints;
extracting a plurality of entities in the plurality of relevant sections;
extracting a plurality of relations between the plurality of entities from the plurality of relevant sections; and
converting the plurality of entities and the plurality of relations into one or more logical statements, wherein the one or more logical statements form the one or more adjudication rules.

11. The system as claimed in claim 8, wherein configuring the plurality of processing models for processing the one or more adjudicable events and the one or more endpoints comprises:
filtering data related to the one or more adjudicable events and the one or more endpoints from the patient clinical information according to the clinical trial master documents;
pre-processing the filtered data;
selecting the plurality of processing models using a pre-defined decision matrix based on a data type of the filtered data and a medical field in which the one or more clinical trials are conducted; and
generating a vector indicating configuration of executing the plurality of processing models for the clinical event and endpoint adjudication.

12. The system as claimed in claim 11, comprising:
determining if the patient clinical information is sufficient or insufficient in accordance with the clinical trial master documents prior to the step of filtering data; and
performing one of: i) proceeding with the step of filtering data if the patient clinical information is sufficient, and ii) obtaining required data if the patient clinical information is insufficient, wherein if the required data is not available, performing:
proceeding with the step of filtering data; and
marking the required data as permanently missing.

13. The system as claimed in claim 8, wherein the clinical trial master documents obtained as input data are compared with one or more previously obtained clinical trial master documents to identify if there are older versions of the clinical trial master documents, wherein upon identification of the older versions of the clinical trial master documents, performing:
identifying one or more changes in the clinical trial master documents; and
generating the adjudication rules associated with the one or more changes.

14. One or more non-transitory machine-readable information storage mediums comprising one or more instructions which when executed by one or more hardware processors cause:
obtaining an input data related to one or more clinical trials and clinical event adjudication, wherein the input data comprises: i) patient clinical information, and ii) clinical trial master documents;
generating one or more adjudication rules from the clinical trial master documents;
identifying one or more adjudicable clinical events and one or more endpoints from the patient clinical information based on the one or more adjudication rules;
determining a configuration of a plurality of processing models for processing the one or more adjudicable events and the one or more endpoints;
executing the plurality of processing models according to the determined configuration using the patient clinical information to obtain a first result of the clinical event adjudication;
obtaining a second result of the clinical event adjudication by processing the one or more adjudicable events and the one or more endpoints using a pre-trained real world evidence inference model;
applying a weighted average technique on the first result of the clinical event adjudication and the second result of the clinical event adjudication to obtain a final result of the clinical event adjudication; and
generating an adjudication report based on the final result of the clinical event adjudication and one or more adjudication forms comprised in the clinical trial master documents.

15. The one or more non-transitory machine-readable information storage mediums as claimed in claim 14, wherein the patient clinical information is obtained from at least one of: i) one or more source documents, and ii) an input feed from one or more clinical data collection systems, and wherein the clinical trial master documents comprise a Clinical Event Committee (CEC) charter, a clinical protocol, and the one or more adjudication forms, wherein the CEC charter outlines one or more procedures, rules, and guidelines for conducting the clinical event adjudication, and wherein the clinical protocol describes one or more objectives, design, methodology, statistical considerations, and aspects related to organization of the one or more clinical trials.
